# EUROPÄISCHE PATENTSCHRIFT

(11) **EP 1 214 589 B1**
(45) Veröffentlichungstag und Bekanntmachung des Hinweises auf die Patenterteilung: **02.06.2004**
(21) Anmeldenummer: 00960653.4
(22) Anmeldetag: 13.09.2000
(51) Int. Cl.: G01N 30/46

(54) **VERFAHREN UND VORRICHTUNG ZUM AUFFINDEN UND ZUR ISOLIERUNG PHARMAKOLOGISCHER VERBINDUNGEN AUS SUBSTANZGEMISCHEN**
METHOD AND DEVICE FOR DETECTING AND ISOLATING PHARMACOLOGICAL COMPOUNDS BEING CONTAINED IN SUBSTANCE MIXTURES
PROCEDE ET DISPOSITIF POUR DETECTER ET ISOLER DES COMPOSES PHARMACOLOGIQUES CONTENUS DANS DES MELANGES DE SUBSTANCES

(30) Priorität: 22.09.1999 DE 19945351
(43) Veröffentlichungstag der Anmeldung: 19.06.2002
(73) Patentinhaber: LTS Lohmann Therapie-Systeme AG, 56626 Andernach (DE)
(72) Erfinder: LENZ, Jana, Institut für Pharmazeutische Chemie, 35032 Marburg (DE); MATUSCH, Rudolf, Inst. für Pharmazeutische Chemie, 35032 Marburg (DE); HOFFMANN, Hans, Rainer, 56566 Neuwied (DE)
(74) Vertreter: Flaccus, Rolf-Dieter, Dr.
(86) Internationale Anmeldenummer: PCT/EP2000/008919
(87) Internationale Veröffentlichungsnummer: WO 2001/022078

(56) Entgegenhaltungen:
- WO-A-92/02815
- WO-A-92/02818
- WO-A-92/17259
- WO-A-93/20449
- WO-A-97/01755
- WO-A-99/33862
- US-A- 5 491 096
- ONNERFJORD P ET AL: "High sample throughput flow immunoassay utilising restricted access columns for the separation of bound and free label" JOURNAL OF CHROMATOGRAPHY A,NL,ELSEVIER SCIENCE, Bd. 800, Nr. 2, 27. März 1998 (1998-03-27), Seiten 219-230, XP004113597 ISSN: 0021-9673

## Beschreibung

Die Arzneimittelforschung hat sich, ausgehend von der Nutzung ausschliesslich natürlicher Quellen über die chemische Synthese von Wirkstoffen und deren Prüfung durch Tierversuche zum gezielten computergestützen Strukturdesign von Wirkstoffen mit Hilfe des Einsatzes experimenteller und theoretischer Methoden hin entwickelt.

Mit zunehmender Kenntnis der verschiedenen Krankheitsursachen (z. B. das Fehlen oder die genetisch bedingte Veränderung eines Proteins) ist die Arzneimittelforschung und die Therapie mit Arzneimitteln wesentlich komplexer geworden. So konnten in den vergangenen zehn Jahren mittels molekularbiologischer Methoden (Humanes Genom Projekt) die genetischen Ursachen einiger primär neurodegenerativer Erkrankungen wie des Morbus Alzheimer, des Morbus Parkinson, des Morbus Huntington, der Amyotrophen Lateralsklerose, der Prionkrankheiten und verschiedener ataxischer Syndrome aufgeklärt werden. Dieses Erkennen der den Krankheiten zugrundeliegenden biologischen Veränderungen stellt die Grundlage für einen Wechsel von einer symptomatischen, palliativen hin zu einer kausalen Therapie dar.

100 bis 150 der circa 30.000 in der Medizin beschriebenen Krankheiten sind so relevant, dass sie sich als Forschungsprojekte für die Pharmaindustrie eignen. Die derzeit zur Verfügung stehenden Medikamente zielen auf eine therapeutische Beeinflussung von ca. 400 Rezeptoren, Enzymen und anderen Biomolekülen. Man geht jedoch davon aus, dass etwa bis zu 10.000 Gene und deren Produkte als Target für die Wirkstoffforschung in Frage kommen. Der Nachweis ihrer pathologischen Relevanz erfordert unter anderem aussagekräftige molekulare und zelluläre Systeme.

Neben dem rationalen Design, das die Optimierung von Stoffeigenschaften aufgrund von Erfahrungswerten oder basierend auf bekannten molekularen Strukturen einbezieht, spielen derzeit die Kombinatorische Chemie und die in der Entwicklung befindliche Kombinatorische Biosynthese eine grosse Rolle in der Arzneimittelforschung.

Eine bedeutende Schwachstelle dieser Methoden ist die eingeschränkte Diversität synthetischer Substanzen, verglichen mit der strukturellen Komplexität pflanzlicher und mikrobieller Sekundärmetabolite.

Um diese natürliche Vielfalt erschliessen zu können, ist eine enge Verknüpfung der klassischen Naturstoffforschung mit der molekularen Medizin und der organischen Chemie unumgänglich. Auf der Suche nach neuen Leitstrukturen erfolgt die Auswahl pflanzlicher und tierischer Organismen sowie der Pilze und Mikroorganismen nach dem Zufallsprinzip, unter chemotaxonomischen Gesichtspunkten, aufgrund ökologischer Beobachtungen und aufgrund ethnomedizinischer Vorkenntnisse.

Das Auffinden einer oder mehrerer wirksamer Komponente(n) aus Substanzgemischen wie z. B. aus durch Kombinatorische Chemie erstellten Substanzbibliotheken oder aus Naturstoffextrakten ist jedoch sehr aufwendig.

Naturstoffextrakte z. B. bestehen im allgemeinen aus einer Vielzahl (bis zu 2.000) unterschiedlichster, den gesamten Polaritätsbereich umfassenden Substanzen, was durch verschiedenartige Grundstrukturen und funktionelle Gruppen bedingt ist. In der Regel machen hier nur relativ wenige Verbindungen bereits ca. 80 % des Extraktgewichtes aus, während die Überzahl der restlichen Verbindungen jedoch in geringer Konzentration bis hin zum ppm-Bereich, also nicht-equimolar vorliegen. Häufig zeigen in einem solchen Extrakt aber nur wenige oder sogar nur eine einzige Substanz die charakteristische biologische Aktivität, wobei diese Aktivität auf eine im Extrakt in Spuren vorliegende Substanz zurückzuführen sein kann.

Bisher erfolgt das Aufarbeiten und Analysieren der meist chromatografisch getrennten Inhaltsstoffe eines natürlichen Extrakts oder einer mittels Kombinatorischer Chemie hergestellten umfangreichen Substanzbibliothek in der Regel unter Anwendung von automatisierten Testsystemen mit extrem hohem Durchsatz (High-throughput Screening; HTS). Dieses Verfahren ist jedoch sehr aufwendig und kostenintensiv. So ist es erforderlich, aus der Naturstoffquelle (z. B. Pflanze, Tier, Pilz, Mikroorganismus) zunächst selektive Extrakte mit Lösungsmitteln steigender Polarität herzustellen und diese biologisch zu testen. Weitere Tests erfolgen nach der Bildung von Unterfraktionen aus dem jeweils wirksamen selektiven Extrakt.

Schliesslich muss ein letzter Test zeigen, welche Reinsubstanz(en) nach Isolierung aus der wirksamen Fraktion biologische Aktivität zeigen und somit einen "Hit" darstellen. Für die chromatografische Auftrennung in Unterbibliotheken und deren Testung werden jeweils mehrere Wochen benötigt. Um ausreichende Mengen der Reinsubstanz(en) gewinnen zu können, muss daher der Start mit grossen Extraktmengen erfolgen. Auch dies ist verbunden mit hohen Kosten für präparative HPLC-Säulen und den hohen Lösungsmittelbedarf (sowohl Anschaffung als auch Entsorgung).

Bereits durch die Auftrennung in Unterfraktionen, aber erst recht durch die Isolierung der reinen Naturstoffe gehen eventuell vorhandene synergistische oder antagonisierende Effekte der einzelnen Komponenten des Extraktes im Hochdurchsatz-Screening verloren. So kann ein im ersten Test wirksamer Extrakt seine biologische Wirksamkeit einbüssen, weil das Auftrennen zu einzelnen Substanzen eine Targetbindung, die nur im Zusammenspiel verschiedener Komponenten möglich war, verhindert.

Ein Verfahren zum Auffinden wirksamer Komponenten aus einer synthetischen, durch Kombinatorische Chemie erstellten Peptidbibliothek, die aus maximal 19 chemisch sehr ähnlichen, sich nur durch Austausch von Aminosäuren ergebenden und in equimolaren Mengen vorliegenden Peptiden besteht, wird von Zuckermann et al., *Proc. Natl. Acad. Sci. USA,* 89, 4505-4509 (**1992**) beschrieben. Dazu wurde ein Antikörper im Unterschuss zu einer solchen Peptid-Substanzbibliothek gegeben und der Target (=Antikörper)-Peptid-Komplex durch schnelle Gelfiltration abgetrennt. Das Peptid wird aus dem Komplex mit 1 % Trifluoressigsäure freigesetzt und die Struktur durch Massenspektroskopie und Aminosäureanalyse aufgeklärt. Dieses Verfahren eignet sich aber nicht zur Isolierung von Target-Molekül-Komplexen kleinerer Moleküle (Molekulargewicht kleiner oder gleich 1500), da die Gelfiltration nur mit grösseren Molekulargewichtsunterschieden technisch funktioniert. Auch erfordert es nach Aussage der Autoren equimolare Mischungen. Weiterhin ist die Ermittlung synergistisch wirkender Kombinationen von Liganden unmöglich bzw. dem Zufall überlassen.

Auch die von Wieboldt et al. in *Anal. Chem.,* 69, 1683-1691 **(1997)** beschriebenen Experimente sind ebenfalls auf equimolare Gemische von 20 bis 30 eng verwandte Moleküle (synthetisch hergestellte Derivate mit einer allgemeinen 1,4-Benzodiazepin-Struktur) gerichtet. Die eingeschränkte Diversität der synthetischen Substanzen erleichtert zwar die experimentelle Bearbeitung, stellt aber zugleich einen begrenzenden Faktor für ihre Anwendbarkeit dar.

Ebenso benötigt die von R. B. van Breemen et al. in *Anal. Chem.,* 69,2159-2164 **(1997)** beschriebene gepulste Ultrafiltration Massenspektrometrie eine equimolare Substanzbibliothek mit 20 Substanzen. Da nur mit organischen Lösemitteln freigesetzt wird, können kovalent gebundene Substanzen nicht entdeckt werden.

Die Aufgabe der vorliegenden Erfindung ist daher die Entwicklung einer schnell durchführbaren und effizienten Methode zum Auffinden und zur Isolierung von biologisch, z. B. pharmakologisch aktiven chemischen Substanzen und Substanzkombinationen, insbesondere aus nicht equimotaren Gemischen wie Naturstoffextrakten (z. B. aus Pflanze, Tier, Pilz, Mikroorganismus).

Die Aufgabe wird durch ein Verfahren gelöst, das durch folgende Schritte gekennzeichnet ist:
a) Hinzufügen eines Targets zu einem Gemisch von chemischen Substanzen, z. B. einem Naturstoffextrakt,
b) Bildung mindestens eines Komplexes aus Target und mindestens einer aktiven chemischen Substanz, wobei diese chemische Substanz an das Target gebunden wird,
c) Abtrennung der nicht gebundenen chemischen Substanzen des Gemisches von dem mindestens einen Komplex und ggf. deren analytische (z. B. chromatografische) Erfassung (= Hauptversuch).

Die in einem separaten Versuch ohne Target (Blindprobe) zu ermittelnden, zusätzlich oder in höheren Konzentrationen auftretenden Substanzen stellen die Summe aller vom Target gebundenen Substanzen dar. Diese werden isoliert und strukturell aufgeklärt.

Als weitere Schritte zur Aufarbeitung des Komplexes können aber auch die:
d) Freisetzung der im Komplex an das Target gebundenen mindestens einen aktiven chemischen Substanz unter Zerstörung der Bindung zwischen aktiver chemischer Substanz und dem Target im Komplex, die
e) Abtrennung der mindestens einen freigesetzten aktiven chemischen Substanz und die
f) Identifizierung der mindestens einen abgetrennten aktiven chemischen Substanz und ggf. der Vergleich mit den im Schritt c) identifizierten Substanz(en)
in Frage kommen.

Unter einem biologischen Target versteht man ein Protein (z. B. Rezeptor, Enzym, Antikörper), eine biologische Membran oder eine ganze (gesunde oder Krebs-)Zelle. Bei Kontakt, insbesonders bei Bindung zwischen einer passenden aktiven chemischen Substanz mit diesem Target kann die Auslösung einer Reaktion erfolgen, die für das Target charakteristisch ist und meist mit einem biochemischen Prozess in Verbindung steht. Mit anderen Worten: Die aktive chemische Substanz besitzt eine starke Affinität zu dem speziellen Target. Beispiele für solche Targets sind die Proteine Thrombin, Trypsin und der β₂-Adrenorezeptor.

Als "chemische Substanz" kommen praktisch alle aus der organischen und Naturstoffchemie bekannten, einheitlichen Stoffe in Frage. Hierzu zählen niedermolekulare und hochmolekulare chemische Stoffe, nicht jedoch Polymere aus einer unbekannten Zahl von Monomereinheiten. Dem Fachmann sind solche chemisch einheitlichen Stoffe der organischen Chemie bekannt. Hierzu zählen aliphatische, aromatische und cyclische Kohlenwasserstoffe und Verbindungen mit funktionellen Gruppen, wie Carbonsäuren, Alkohole, Ester, Aldehyde, Lactone, Amide, Heterocyclen, Isoprenoide, Terpene, Kohlenhydrate, Steroide etc. Auch Glykoside, Peptide, Proteine und Enzyme können als eine geeignete chemische Substanz in Frage kommen.

Die Molekülmassen geeigneter chemisch einheitlicher Substanzen liegen im allgemeinen oberhalb von Mᵣ = 150. So besitzen beispielsweise die bekannten Neurotransmitter Acetylcholin [H₃CCO-O-CH₂-CH₂-N(CH₃)₃]OH und Nicotin Molekülmassen von Mᵣ = 163 bzw. Mᵣ = 162 und beschreiben somit praktisch die Untergrenze der Molekülmasse der geeigneten chemischen Substanzen. Die Obergrenze der Molekülmasse ist prinzipiell nicht beschränkt. So sind hochmolekulare Proteine (Mᵣ bis etwa 300.000) aufgrund ihrer spezifischen Reaktion mit ganz speziellen biologischen Targets durchaus interessante aktive chemisch einheitliche Substanzen, die durch das erfindungsgemässe Verfahren aus einem Gemisch verschiedener chemischer Substanzen abgetrennt werden können.

Uneinheitliche Biopolymere wie z. B. Glykogen, Cellulose etc. kommen für das erfindungsgemässe Verfahren nicht in Frage kommen, weil sie wegen einer undefinierten Zahl von Monomereinheiten keine chemisch einheitlichen Substanzen im Sinne der vorliegenden Erfindung darstellen.

Bei den chemischen Substanzen ist weiterhin zwischen aktiven und inaktiven chemischen Substanzen zu unterscheiden. Unter einer aktiven chemischen Substanz ist eine solche zu verstehen, die befähigt ist, bei Bindung zu einem speziellen Target eine dafür charakteristische Reaktion auszulösen. Eine aktive chemische Substanz ist dadurch charakterisiert, dass sie eine Affinität zum Target besitzt. Eine inaktive chemische Substanz braucht keine Affinität zum Target zu besitzen.

Eine echte Obergrenze der Molmasse der in Frage kommenden chemischen Substanz kann daher nicht beziffert werden. Vielmehr wird nur die Einfachheit der Aufarbeitung im wesentlichen durch das Verhältnis der Molmasse der aktiven chemischen Substanz zur Molmasse des Targets festgelegt. Die Molmasse dieses Targets ist im allgemeinen sehr hoch, und kann z. B. oberhalb von 1 Million liegen. Wie gesagt kann das Target auch eine ganze Zelle sein. Chemische Substanzen mit kleiner Molmasse (etwa 150 bis etwa 30.000) sind von solchen Targets aufgrund der grossen Massendifferenz leicht durch konventionelle Verfahren wie Ultrazentrifugation abzutrennen. Wenn aber die Molmasse der aktiven chemischen Substanz und die Molmasse des Targets in vergleichbaren Grössenordnungen liegen, liefert auch die Ultrazentrifugation nur noch unbefriedigende Trennergebnisse und man muss anspruchsvollere oder zusätzliche Trennverfahren anwenden.

Unter einem "Gemisch von chemischen Substanzen" ist eine Mischung von verschiedenen chemischen Substanzen zu verstehen, die mindestens eine aktive chemische Substanz enthält, die das oben genannte Kriterium erfüllt, d. h. die eine Reaktion bei Kontakt an einem speziellen Target auszulösen vermag. Das Gemisch kann auch mehrere solcher aktiver chemischer Substanzen enthalten. Weiterhin kann das Gemisch auch chemische Substanzen enthalten, die keine Reaktion an einem biologischen Target auszulösen vermögen. In der Regel stellen die inaktiven chemischen Substanzen den Hauptanteil in dem Gemisch verschiedener chemischer Substanzen dar. Insbesondere sind die im Gemisch von chemischen Substanzen enthaltenen inaktiven chemischen Stoffe nur in Bezug auf ein speziell ausgewähltes Target inaktiv, sind aber durchaus in der Lage, an einem anderen Target eine solche Reaktion auszulösen.

In der Praxis handelt es sich bei dem Gemisch chemischer Substanzen bevorzugt um Substanzbibliotheken, die synthetisch oder mit Hilfe der Kombinatorischen Chemie hergestellt werden oder um einen Naturstoffextrakt. Unter einem Naturstoffextrakt im Sinne dieser Definition sind somit komplexe Gemische chemisch einheitlicher Substanzen zu verstehen, die aus einer biologischen Quelle stammen und vorzugsweise aus Pflanzen, Pflanzenteilen wie Blättern, Blüten, Holz, Wurzeln, Rinde etc., Pilzen, Tieren, Drüsen, Eiern und Exkrementen von Tieren, Mikroorganismen etc. gewonnen werden. Dies geschieht durch bekannte Verfahren, z. B. Wasserdampfdestillation, trockene Destillation, Extraktion mit Wasser, organischen, anorganischen oder überkritischen Lösungsmitteln; häufig auch unter gleichzeitiger bzw. artschliessender chemischer Weiterverarbeitung wie Veresterung, Verseifung, Salzbildung, Hydrierung, Dehydratation, Isomerisierungen, Alkylierungen, Fermentation, enzymatischer Abbau etc. Die so gewonnenen Naturstoffextrakte entsprechen also, was die Zusammensetzung ihrer Inhaltsstoffe anbetrifft, mitunter nicht mehr der in der biologischen Quelle vorliegenden Zusammensetzung. Im allgemeinen sind aber eine Vielzahl, d. h. mindestens 50 unterschiedlichster chemischer Substanzen vorhanden, darunter - wie bereits gesagt - zahlreiche nur in Spuren, d. h. in einer Konzentration von nur einigen ppm. Häufig zeigen in einem solchen Extrakt aber nur wenige oder sogar nur eine einzige Substanz die charakteristische biologische Aktivität, wobei diese Aktivität auf eine im Extrakt nur in Spuren vorliegende Substanz zurückzuführen sein kann. Das Gemisch chemischer Substanzen kann auch ein Gemisch verschiedener Naturstoffextrakte sein. im konkreten Beispiel wurde ein Extrakt aus Löwenzahn (Taraxacum officinale) gewählt. Besonders wichtige biologische Quellen stellen natürlich die Heilpflanzen dar, deren Extrakte physiologische und / oder pharmakologische Wirkungen besitzen und zum Teil im DAB und HAB genannt werden.

Das "Hinzufügen" des Targets zu dem Gemisch von chemischen Substanzen erfolgt vorzugweise in Lösung, Suspension oder Dispersion. In vielen Fällen kann das Hinzufügen in einer wässrigen Lösung, insbesondere in einer, deren pH-Wert mit Hilfe eines geeigneten Puffers stabilisiert wird, erfolgen. Es ist ein besonderer Vorteil der hier beschriebenen Methode, dass eine vorangehende Auftrennung des Gemisches von chemischen Substanzen in mehrere verschiedene Fraktionen bis zu reinen chemischen Substanzen vor dem Hinzufügen des Targets nicht erfolgt.

Mit einem "Komplex" ist ein isolierbares Teilchen gemeint, welches aus dem Target besteht, an das mindestens eine aktive chemische Substanz gebunden ist. Ein Target kann auch zwei oder mehrere aktive chemische Substanzen an sich binden. Die in einem solchen Komplex an das Target gebundenen zwei oder mehr aktiven chemischen Substanzen können in einem bestimmten, charakteristischen Verhältnis zueinander vorliegen, welches hinsichtlich der spezifischen Reaktion des speziellen Targets einer synergistischen Wirkung entspricht. Der Fachmann spricht wegen der häufigen Konstellation, dass als Target ein Protein gewählt wird, auch von Protein-Ligand-Komplexen.

Im Komplex ist die mindestens eine aktive chemische Substanz an das Target "gebunden". Dabei spielt die Art der Bindung zwischen Target und der oder den aktiven chemischen Substanz(en) grundsätzlich keine Rolle. Am häufigsten treten kovalente oder nicht-kovalente Bindungen auf. Zu letzteren zählen z. B. Wasserstoffbrücken, elektrostatische Wechselwirkungen, z. B. zwischen entgegengesetzt geladenen Gruppen, Metallkomplexierung, Wechselwirkungen von lipophilen Gruppen der aktiven chemischen Substanz mit hydrophoben Bereichen (sog. Taschen) des Targets, Dipol-Dipol-Wechselwirkungen und Kation-TT-Wechselwirkungen. Es ist auch oft die Kombination verschiedener Wechselwirkungen, die die Affinität einer aktiven chemischen Substanz zum Target verursachen.

Die "Abtrennung" des mindestens einen Komplexes von den nicht gebundenen, d. h. den "freien" inaktiven chemischen Substanzen des Gemisches erfolgt grundsätzlich mit üblichen Methoden, wobei besonders vorteilhaft Verfahren ohne thermische Belastung des Komplexes gewählt werden. Dazu zählen Filtration, Ultrafiltration, Zentrifugation, Ultrazentrifugation, Gleichgewichtsdialyse, Gelfiltration oder Präzipitation des Komplexes. Die Identifizierung der mindestens einen an das Target gebundenen "aktiven" chemischen Substanz kann mitunter vor der Freisetzung dieser chemischen Substanz aus dem Komplex, z. B. mit Flugzeitmassenspektroskopie (MALDI-TOF) erfolgen.

Die Filtration stellt eine besonders schnelle, einfache und effiziente Separationsmethode dar. Sie kann als Ultrafiltration (z. B. unter Anwendung von Microcon-Filtereinheiten der Firma Amicon) oder mit speziellen Filtrationsgeräten (z. B. Brandel-Zellsammler) durchgeführt werden.

Die "Freisetzung" der mindestens einen aktiven chemischen Substanz aus dem Komplex erleichtert jedoch ihr Auffinden, Isolieren und insbesondere ihre chemische Charakterisierung. Sie schliesst sich dem Trennungsschritt nach zwei oder mehr Waschgängen zur Entfernung unspezifisch adsorbierter Substanzen an. Es wird dadurch die Freisetzung der gebundenen aktiven chemischen Substanzen aus dem Komplex erzielt. Dabei wird die Bindung, die im Komplex zwischen Target und der mindestens einen aktiven chemischen Substanz besteht, wieder gelöst. Man verwendet dazu - abhängig von der Natur der Bindung - physikalische oder chemische Methoden. Das kann z. B. mittels einer sauren, wässrig-niederalkanolischen Lösung, bevorzugt mit einem Gemisch von Trifluoressigsäure/Methanol/Wasser, z. B. der Zusammensetzung 1/49,5/49,5 (Vol.-%), erfolgen.

Man kann auf den Freisetzungsschritt verzichten, wenn eine Identifizierung der aktiven chemischen Substanz nicht erforderlich ist bzw. wenn der Komplex als solcher identifiziert wird. Man muss auf die Freisetzung verzichten, wenn sich die aktive Substanz durch die Freisetzungsbedingungen verändert werden würde oder sich nicht durch die Freisetzungslösung abtrennen lässt. Dies ist hauptsächlich bei einigen kovalenten Bindungen der Fall. In diesen Fällen erfolgt die Identifizierung der aktiven chemischen Substanz aus der Differenz der Filtrate mit Target (= Hauptversuch) und ohne Target (Blindprobe). Dieses Verfahren erlaubt es, alle vom Target gebundenen chemischen Substanzen gleichzeitig zu erfassen, während die Freisetzung in der Regel nur wenige (z. B. eins bis drei) aktive chemische Substanzen ergibt, wobei man zudem nicht sicher sein kann, dass sie unverändert sind.

Die in der Blindprobe zusätzlich oder in höherer Konzentration auftretenden Substanzen stellen die potentiell aktiven chemischen Substanzen dar. Sie können mit einer beliebigen Trennmethode (z. B. chromatografisch) auch in grösseren Mengen aus dem Gemisch chemischer Substanzen (z. B. dem Naturstoffextrakt) isoliert werden.

Die "Abtrennung" der mindestens einen freigesetzten aktiven chemischen Substanz von dem Target nach Zerstörung der im Komplex bestehenden Bindung kann mit denselben, grundsätzlich bekannten Methoden erfolgen, die bereits genannt wurden. Auch hier werden bevorzugt die einfachsten und zweckmässigsten Methoden angewandt, d. h. Filtration und Zentrifugation. Die Abtrennung der aktiven chemischen Substanz(en) kann auch mittels präparativer HPLC erfolgen.

Die "Identifizierung" der in der Blindprobe zusätzlich oder in höherer Konzentration auftretenden Substanzen und der mindestens einen abgetrennten aktiven chemischen Substanz erfolgt durch übliche Methoden, wie HPLC, z. B. konventionelle analytische HPLC, Micro-HPLC, Kapillar-HPLC oder Nano-HPLC, oder durch Elektrochromatografie, Elektrophorese oder Kopplungstechniken von LC-MS oder MS-MS. Die Identifizierung der aktiven chemischen Substanz(en) mittels der genannten Methoden kann allerdings auch bereits vor ihrer Freisetzung aus dem Komplex erfolgen.

Das erfindungsgemässe Verfahren unterscheidet sich grundsätzlich vom bekannten Hochdurchsatz-Screening, und zwar im wesentlichen dadurch, dass durch das gezielte Bilden eines Komplexes aus Target und mindestens einer aktiven chemischen Substanz und dem nachfolgenden Abtrennen der nichtgebundenen inaktiven chemischen Substanzen (Hauptversuch) und der Differenz zur Blindprobe mittels dieses geeigneten speziellen Targets eine einzige oder einige wenige aktive chemische Substanz(en), die sich an das entsprechende Protein binden (sog. "Liganden"), aus einer Substanzbibliothek oder aus einem komplexen Naturstoffgemisch isoliert und identifiziert werden können.

Anstatt jede potentiell interessante chemische Substanz einzeln zu isolieren und dem Target zuzuführen, präsentiert man dieses dem Substanzgemisch. Die sich nach Erkennung entsprechend Emil Fischers "Schlüssel-Schloss-Prinzip" bildenden Komplexe werden z. B. durch Ultrafiltration von den ungebundenen niedermolekularen chemischen Substanzen abgetrennt und durch vergleichende Chromatografie (targetfreie [= Blindprobe] versus targethaltige [= Hauptversuch] Probe) identifiziert. Aus den Komplexen werden dann zur Bestätigung der Ergebnisse die Liganden (die aktiven chemischen Substanzen) zusätzlich freigesetzt - soweit diese stabil und freisetzbar sind -, durch übliche Methoden identifiziert, isoliert und mit den gängigen Methoden der Analytik strukturell aufgeklärt.

Das erfindungsgemässe Verfahren erfordert weder grosse Protein- noch Extraktmengen, benötigt durch Downscaling auf Mikromethoden in der Analytik geringe Lösungsmittelvolumina, erfordert keine zeitintensive Unterfraktionierung, und ermöglicht die Entdeckung synergistisch wirkender Substanzkombinationen. Auf Radioaktiv- und Fluoreszenzmarkierungen kann verzichtet werden. Das erfindungsgemässe Verfahren ermöglicht natürlich auch die Aufarbeitung grösserer Extraktmengen, wobei aktive chemische Substanzen "gefischt" werden können, deren Eigenschaften für die Weiterverwendung dieses Extraktes unerwünscht sind.

Besonders vorteilhaft kann das Verfahren eingesetzt werden, wenn aus grösseren Mengen solcher Extrakte nur die aktiven chemischen Substanzen "gefischt" werden sollen, die eine Wechselwirkung mit dem betreffenden Target zeigen. Die mindestens eine abgetrennte aktive chemische Substanz kann dann anstelle des Naturstoffgemisches als wirksames Prinzip eines Arzneimittels weiterverwendet werden. Dies ist auch dann von Bedeutung, wenn mehr als eine aktive chemische Substanz des Naturstoffextraktes mit dem Target einen Komplex bildet und das relative Verhältnis der mehr als einen aktiven chemischen Substanz zueinander für die biologische Wirksamkeit des Naturstoffextrakts eine dominierende Rolle spielt (Synergieeffekt).

Ein Vorteil dieses Verfahrens liegt somit auch in der Möglichkeit zur Identifizierung von wirksamen (synergistischen) Substanzkombinationen, die beim Hochdurchsatz-Screening von Einzelsubstanzen nicht zugänglich sind.

Schliesslich konnte mit Hilfe dieses Verfahrens auch die Erkennungsgrenze (Nachweisgrenze) für aktive chemische Substanzen wider Erwarten in den mikromolaren Kᵢ-Wert-Bereich hinein verschoben werden; d. h. Komplexe aus einem Target und einer aktiven chemischen Substanz mit einem Kᵢ-Wert von 1,7 µM waren nachweisbar.

Die folgenden Beispiele dienen der anschaulichen Darstellung des erfindungsgemässen Verfahrens.

Beispiel 1: Isolierung eines Thrombinhemmstoffes vom 4-Amidinophenylalanin-Typ aus einer willkürlich ausgewählten Substanzbibliothek.

Als Target wurde die Serinprotease Thrombin gewählt. Als Substanzen der Substanzbibliothek (des Gemisches "inaktiver" chemischer Substanzen im Sinne der vorigen Definition) wurden die folgenden fünf Arzneistoffe ausgewählt und zwar unter Berücksichtigung ihrer Wasserlöslichkeit, ihrer Absorptionsmaxima und ihrer chromatografischen Trennbarkeit:
1. der zentral angreifende α₂-Adrenorezeptor-Agonist Clonidin-HCl
2. das Mucolytikum Bromhexin-HCl
3. das tricyclische Antidepressivum Amitryptilin-HCl
4. das Neuroleptikum vom Phenothiazin-Typ Chlorpromazin-HCl
5. das Neuroleptikum vom Phenothiazin-Typ Chlorprotixen-HCl

Als Thrombinhemmstoff (die aktive chemische Substanz) wurde die Verbindung CRC 220 der Behringwerke (Marburg, Kᵢ = 2,5 nM) mit der folgenden Strukturformel verwendet:

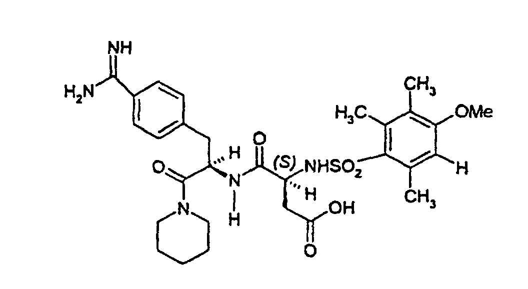

Die untersuchten Assays hatten folgende Zusammensetzung:

**Tabelle 1**

| **Probe** | **Blindprobe ohne Thrombin** | **Hauptversuch mit Thrombin** |
|---|---|---|
| Thrombin 2000 E/mg | 0 | 1 nmol 5 µmol/l |
| Clonidin-HCl | 2 nmol 10 µmol/l | 2 nmol 10 µmol/l |
| Bromhexin-HCl | 2 nmol 10 µmol/l | 2 nmol 10 µmol/l |
| Amitriptylin HCl | 2 nmol 10 µmol/l | 2 nmol 10 µmol/l |
| Chlorpromazin-HCl | 2 nmol 10 µmol/l | 2 nmol 10 µmol/l |
| Chlorprothixen-HCl | 2 nmol 10 µmol/l | 2 nmol 10 µmol/l |
| CRC 220 | 2nmol 10 µmol/l | |
| 0,9 % NaCl in Wasser (reinst) | ad 200 µl | ad 200 µl |

Die Inkubation der Substanzbibliothek und des Inhibitors mit Thrombin erfolgte innerhalb 1 Stunde bei Raumtemperatur; Lösungsmittel: 0,9 % NaCl in H₂O. Die Abtrennung der gebildeten Protein-Ligand-Komplexe erfolgte durch Ultrafiltration (zentrifugal). Alle Filtrationsvorgänge bzw. Waschungen wurden bis zur Trockenheit der Filter durchgeführt.
Filter: Microcon 10 (Amicon)
Zentrifugationsbedingungen: 9981xg, Raumtemperatur
Waschschritte: 2 x mit je 150 µl 0,9 % NaCl in H₂O, 4 °C

Nach der Ultrafiltration und Analyse des Filtrats folgten Waschschritte und die Freisetzung des Liganden (der aktiven chemischen Substanz) aus dem auf dem Filter verbliebenen Protein-Ligand-Komplex durch Behandlung mit 200 µl Wasser/Methanol/TFA (49,5/49,5/1) bei Raumtemperatur.

Die Blindprobe (ohne Thrombin) wurde in allen Schritten analog behandelt, so dass ein Vergleich der Filtrate möglich ist. Die bei allen Schritten erhaltenen Filtrate wurden mittels Speed-VAC Concentrator getrocknet und später im entsprechenden HPLC-Fliessmittel definierter Menge unter Anwendung von Ultraschall und Schütteln gelöst.

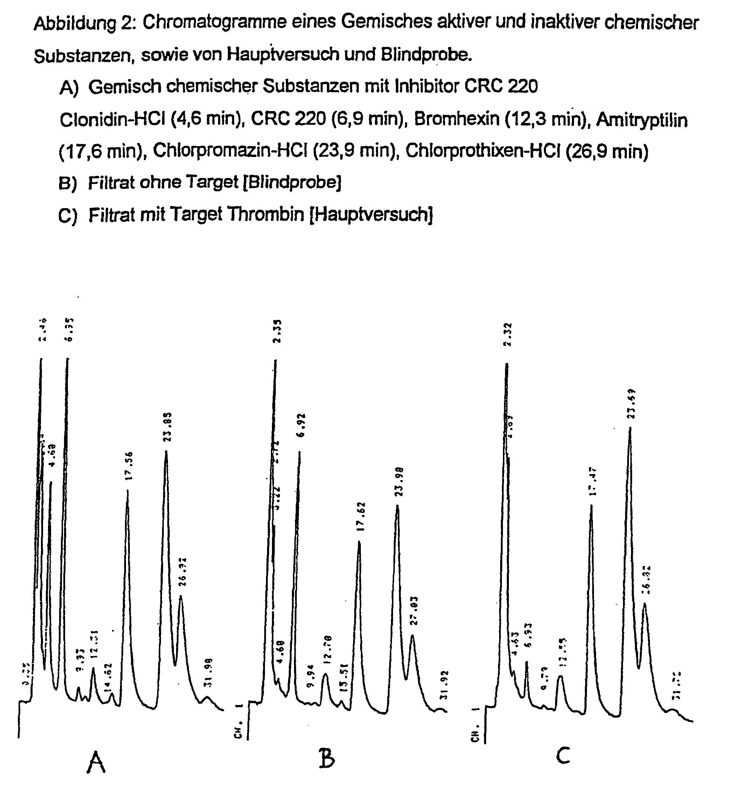

Die Identifizierung der Liganden erfolgte durch analytische HPLC: stationäre Phase Hypersil C 18 BDS, 3 µm, 150 * 0,3mm; Fusica (LC Packings) - mobile Phase: Acetonitril/Wasser/TFA (35/65/,01), isokratisch, 5 µl/min, λ =230 mm. Die Ergebnisse sind in Fig. 2 dargestellt.

Die Differenz zwischen Hauptversuch und Blindprobe ist CRC 220 als aktive chemische Verbindung. Da Clonidin-HCl (4,6 min) weder im Hauptversuch noch in der Blindprobe auftritt, wird es nicht vom Target, sondern vom Filter gebunden. Es ist kein inhibitor.

Beispiel 2: Bindung nicht equimolarer Gemische von Amidinophenylalaminen unterschiedlicher Bindungsstärke an Trypsin.

Es wurden die im folgenden durch ihre Strukturformeln wiedergegebenen Trypsin-Hemmstoffe von 3-Amidinophenylalamin-Typ als chemische Substanzen und die Serinprotease Trypsin als Target verwendet:

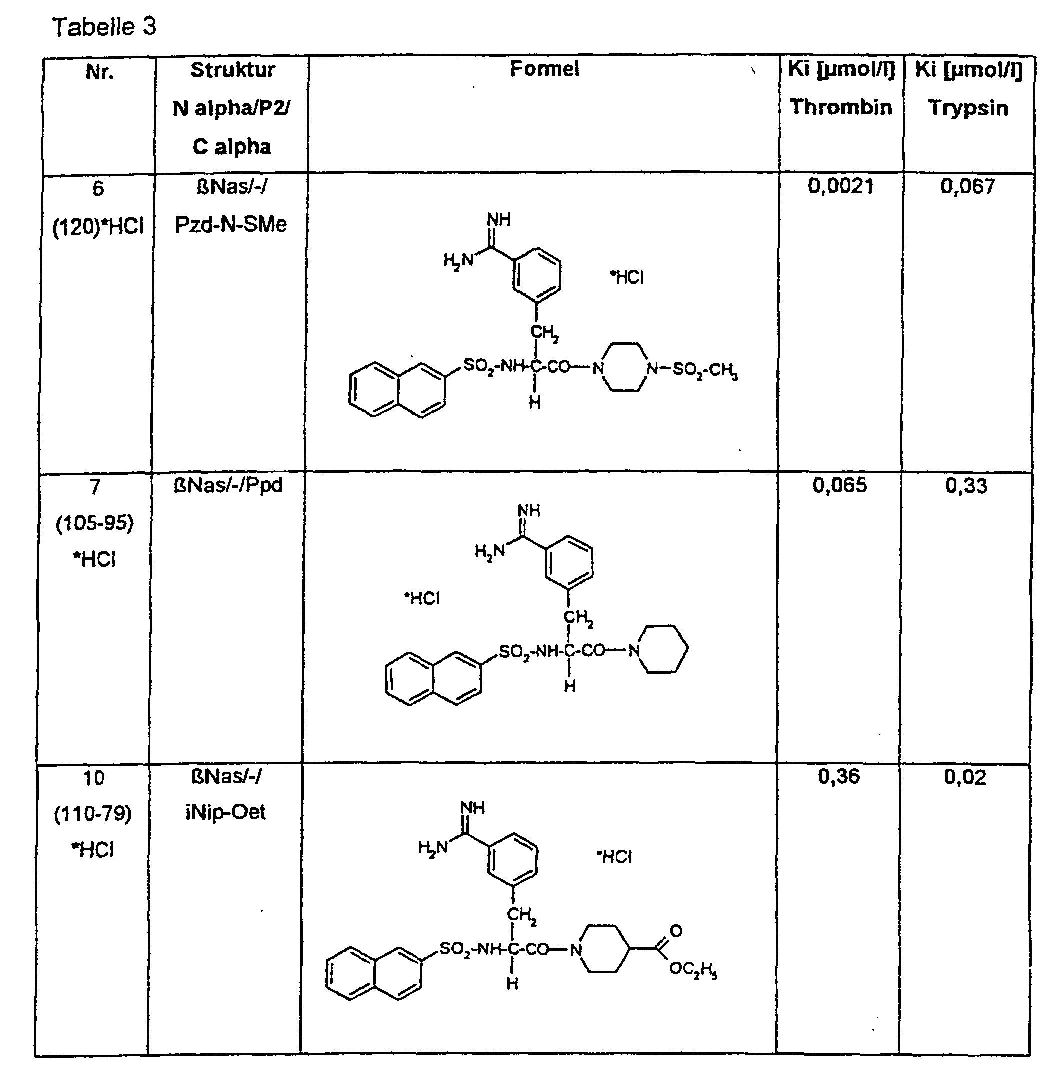

**Tabelle 4:**

| Zusammensetzung der Assays | | | | | |
|---|---|---|---|---|---|
| **Probe** | **Trypsin 10.600 E/mg** | **6 (K**_{**i**}**=67 nM)** | **7 (K**_{**i**}**=330 nM)** | **10 (K**_{**i**}**=20 nM)** | **0,9 % NaCl in Wasser (reinst)** |
| 261; 300 | 0 | 1,68 nmol 8,4 µmol/l | 8,25 nmol 41,25 µmol/l | 0,5 nmol 2,5 µmol/l | ad 200 µl |
| 262.301:302 | 10 nmol 50 µmol/l | 1,68 nmol 8,4 µmol/l | 8,25 nmol 41,25 µmol/l | 0,5 nmol 2,5 µmol/l | ad 200 µl |

Die Versuche wurden durchgeführt wie in Beispiel 1 beschrieben.

**Tabelle 5:**

| Ergebnisse | | | | | |
|---|---|---|---|---|---|
| **Probe** | **Substanz** | **Filtrat [µmol/l]** | **1. Waschung [µmol/l]** | **2. Waschung [µmol/l]** | **Freisetzung [µmol/l]** |
| 261 | 6 | 7,44 | 0,71 | 0,05 | 0,01 |
| | 7 | 31,57 | 3,13 | 0,26 | 0,13 |
| | 10 | 0,05 | 0,02 | 0,0013 | 0,0011 |
| 262 | 6 | 0,67 | 0,23 | 0,15 | 6,09 |
| | 7 | 22,22 | 3,68 | 1,29 | 8,49 |
| | 10 | 0,02 | 0,0058 | 0,0012 | 0,53 |

### Beispiel 3: Isolierung und Identifizierung von Naturstoffen aus Taraxacum-Extrakt

Für die Assays verwendete Substanzen:
a) Extr. Taraxaci spir. sicc. (Naturstoff-Trockenextrakt der Firma Caelo) Herstellung von wässrigen Lösungen:
   - Suspension der Trockenextrakte in Wasser (0,2 g in 20 ml)
   - 5 min Behandlung im Ultraschallbad, 30 min Stehen unter gelegentlichem Schütteln
   - Filtration durch Membranfilter (0,7 µm), anschliessend durch Anotop 25-Filter (0,02 µm)
   - Prüfung auf Gerbstoffe mittels FeCl₃, Pb(CH₃COO)₄ und Gelatine: negativ
b) β₂ Adrenorezeptor (= Target)
   - Membranpräparation aus Sf9-Insektenzellen, die 3 Tage mit einem rekombinanten β₂-adrenergen-Rezeptor-Baculovirus infiziert worden waren (Zellen und Viren, Plasmidkonstruktion, Isolierung des rekombinanten Baculovirus und Praparation der Membranen: MPI für Biophysik, Abt. für Molekulare Membranbiologie, Frankfurt/Main, Dr. Helmut Reiländer) [H. Reiländer, *Febs letters,* 282, 441-444 (1991)]

In den Assays wurden der Taraxacum-Extrakt und die Membranpräparation in unterschiedlichen stöchiometrischen Verhältnissen, gelöst in 200 µl Bindungspuffer (150mM NaCl, 50mM Tris, pH 8,2 in Wasser) kombiniert.

Die Rezeptor-Ligand-Bindung wurde vervollständigt durch 30 Minuten lange Inkubation der Mischung bei 30-34 °C. Die Lösungen wurden dann auf ein Microcon 10-Zentrifugalfilter gegeben und bei 9981xg 15 min lang bzw. bis zur Trockenheit des Filters zentrifugiert. Der Vergleich der Chromatogramme der Proben mit (= Hauptversuch) und ohne Rezeptor (= Blindprobe) führte zur Identifizierung der gebundenen chemischen Substanzen. Nach zwei Waschschritten mit Bindungspuffer wurden die auf dem Filter befindlichen Komplexe getrennt durch Behandlung mit 200 µl TFA (1/49,5/49,5).

**Tabelle 6:**

| Zusammensetzung der Assays: | | | |
|---|---|---|---|
| **Probe** | **β**_{**2**}**-adrenerger Rezeptor (Membranpräparation 10/98; 6,5 pmol Rezeptor pro mg Protein)** | **Extr. Taraxaci (10mg/ml wässriger Extrakt, filtriert durch Anotop 25- Filter (Merck))** | **Bindungspuffer (150mM NaCl; 50mM Tris; pH 8,2)** |
| 351 | 0 | 1,5 mg 7,5 g/l | ad 200 µl |
| 355 | 1,04 pmol 5,2 nmol/l | 1,5 pmol 7,5 nmol/l | ad 200 µl |
| 356 | 1,04 pmol 5,2 nmol/l | 1,5 pmol 7,5 nmol/) | ad 200 µl |
| 360 | 0 | 0,3 mg 1,5 g/l | ad 200 µl |
| 361 | 1,04 pmol 5,2 nmol/1 | 0,3 mg 1,5 g/l | ad 200 µl |
| 362 | 0 | 0,6 mg 3 g/l | ad 200 µl |
| 363 | 1,04 pmol 5,2 nmol/l | 0,6 mg 3 g/l | ad 200 µl |
| 364 | 0 | 1,0 mg 5,0 g/l | ad 200 µl |
| 365 | 1,04 pmol 5,2 nmol/l | 1,0 mg 5,0 g/l | ad 200 µl |
| 366 | 1,04 pmol 5,2 nmol/l | 1,5 mg 7,5 g/l | ad 200µl |
| 367 | 1,04 pmol 5,2nmol/l | 1,5 mg 7,5 g/l | ad 200 µl |
| 369 | 1,04 pmol 5,2 nmol/l | 1,5 mg 7,5 g/l | ad 200 µl |
| 373 | 1,04 pmol 5,2 nmol/l | 1,5 mg 7,5 g/l | ad 200 µl |
| 374 | 0,52 pmol 2,6 nmol/l | 1,5 mg 7,5 g/l | ad 200 µl |
| 375 | 0,26 pmol 1,3 nmol/l | 1,5 mg 7,5 g/l | ad 200 µl |

Die Eluate von den folgenden vier Filtrationsschritten wurden im Vakuum bis zur Trockne zentrifugiert und die erhaltenen Substanzen in 10 µl der mobilen Phase der HPLC (vergl. Beispiel 1) kurz vor der LC-Analyse mittels Ultraschall in Lösung gebracht.

**Tabelle 7:**

| Ergebnisse | | | | | |
|---|---|---|---|---|---|
| **Probe** | **Substanz** | **Filtrat [nmol/l]** | **1. Waschung [nmol/l** | **2. Waschung [nmol/l]** | **Freisetzung [nmol/l]** |
| 351 | L3 | 740,3 | 51,0 | 8,0 | 11,9 |
| | L6 | 5970,7 | 15045 | 750,8 | 195,0 |
| | L7 | 3802,1 | 748,2 | 145,5 | 22,7 |
| 355 | L3 | 353,5 | 88,8 | 12,9 | 15,8 |
| | L6 | 1482,0 | 451,4 | 100,4 | 707,0 |
| | L7 | 1539,6 | 909,9 | 180,4 | 214,5 |
| 356 | L3 | 961,9 | 79,0 | 9,9 | 26,3 |
| | L6 | 1848,6 | 600,1 | 202,1 | 2199,1 |
| | L7 | 2183,1 | 1117,4 | 295,9 | 510,7 |
| 360 | L3 | 411,5 | 22,7 | 0 | 8,1 |
| | L6 | 2935,2 | 468,9 | 140,3 | 34,7 |
| | L7 | 1268,8 | 129,6 | 6,6 | 8,6 |
| 361 | L3 | 537,0 | 16,5 | 29,6 | 11,2 |
| | L6 | 1588,2 | 146,5 | 124,3 | 138,1 |
| | L7 | 1268,8 | 129,6 | 6,6 | 8,6 |
| 362 | L3 | 650,1 | 20,5 | 5,6 | 3,2 |
| | L6 | 3446,6 | 958,0 | 351,4 | 85,4 |
| | L7 | 1423,2 | 176,5 | 33,7 | 7,5 |
| 363 | L3 | 736,9 | 54,4 | 0 | 8,7 |
| | L6 | 1180,6 | 248,3 | 142,0 | 257,0 |
| | L7 | 1740,7 | 76,4 | 104,2 | 16,9 |
| 364 | L3 | 1034,4 | 36,9 | 3,0 | 5,3 |
| | L6 | 3752,4 | 1377,0 | 627,8 | 151,3 |
| | L7 | 1829,6 | 92,7 | 30,1 | 89,6 |
| 365 | L3 | 1221,4 | 51,7 | 8,6 | 9,8 |
| | L6 | 2291,6 | 626,7 | 141,6 | 436,9 |
| | L7 | 2746,8 | 74,0 | 11,2 | 111,9 |
| 366 | L3 | 2088,5 | 102,8 | 2,3 | 7,6 |
| | L6 | 5581,8 | 620,0 | 181,5 | 504,8 |
| | L7 | 4261,6 | 1425,3 | 213,1 | 168,2 |
| 367 | L3 | 2310,8 | 46,2 | 15,6 | 6,3 |
| | L6 | 5159,0 | 832,5 | 274,6 | 616,8 |
| | L7 | 6243,7 | 1509,0 | 199,2 | 232,9 |
| 369 | L3 | 2381,4 | 90,8 | 41,8 | 7,0 |
| | L6 | 7449,7 | 478,1 | 341,9 | 674,0 |
| | L7 | 3505,8 | 986,8 | 221,7 | 378,7 |
| 373 | L3 | 2450,0 | 91,7 | 26,6 | 36,2 |
| | L6 | 5562,6 | 182,7 | 277,1 | 2096,1 |
| | L7 | 4900,2 | 1176,5 | 285,4 | 847,8 |
| 374 | L3 | 2593,2 | 44,0 | 7,4 | 25,3 |
| | L6 | 8589,0 | 1062,4 | 476,6 | 1015,4 |
| | L7 | 5661,6 | 627,7 | 200,9 | 538,8 |
| 375 | L3 | 2252,1 | 39,7 | 19,6 | 19,0 |
| | L6 | 10838,5 | 921,1 | 433,9 | 279,9 |
| | L7 | 4921,4 | 631,3 | 112,3 | 154,4 |

Die drei "gefischten", d. h. durch Komplexbildung und anschliessende Freisetzung aus dem Komplex erhaltenen Liganden (L3 = trans-Caftarinsäure, L6 = trans-Chicorinsäure und L7 = trans-Diferoyl-weinsäureester) konnten mittels UV, ¹H-NMR und MS identifiziert werden.

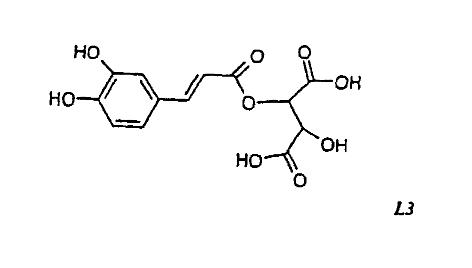

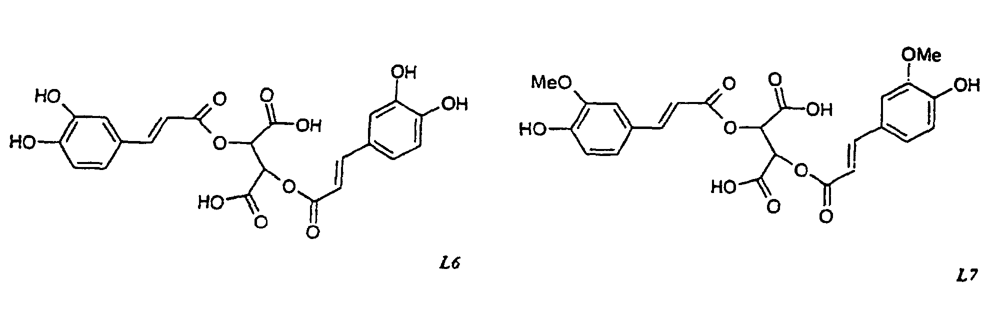

### Beschreibung der Abbildungen

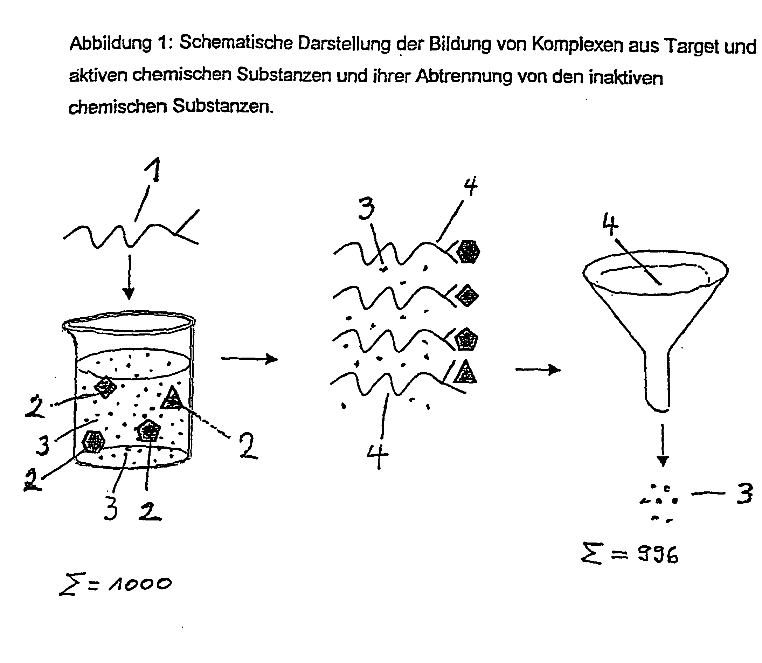

Fig. 1 zeigt eine symbolische Darstellung der Bildung des mindestens einen Komplexes. Zu einem Gemisch von 1000 verschiedenen, nicht equimolaren chemisch einheitlichen Substanzen (davon vier aktiven) wird ein Target hinzugefügt. Es entstehen vier verschiedene Komplexe, die jeweils eine der aktiven chemischen Substanz gebunden enthalten. Die 996 inaktiven Substanzen können dann mittels Ultrafiltration von den Komplexen abgetrennt werden. Es bedeuten:
- 1 =: Target
- 2 =: diverse aktive chemische Substanzen
- 3 =: diverse inaktive chemische Substanzen (Punkte)
- 4 =: verschiedene Komplexe mit mindestens einer aktiven chemischen Substanz

Die Fig. 2 zeigt:
A) Gemisch chemischer Substanzen mit Inhibitor CRC 220 Clonidin-HCl (4,6 min), CRC 220 (6,9 min), Bromhexin (12,3 min), Amitryptilin (17,6 min), Chlorpromazin-HCl (23,9 min), Chlorprothixen-HCl (26,9 min)
B) Filtrat ohne Target [Blindprobe]
C) Filtrat mit Target Thrombin [Hauptversuch]

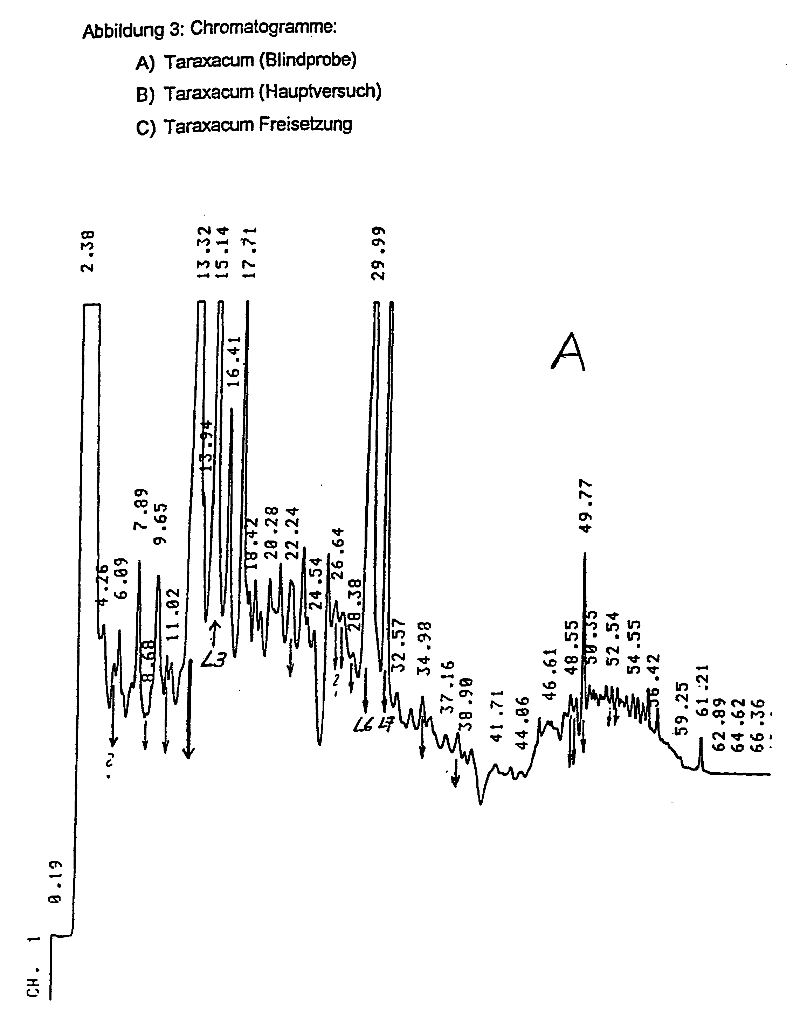

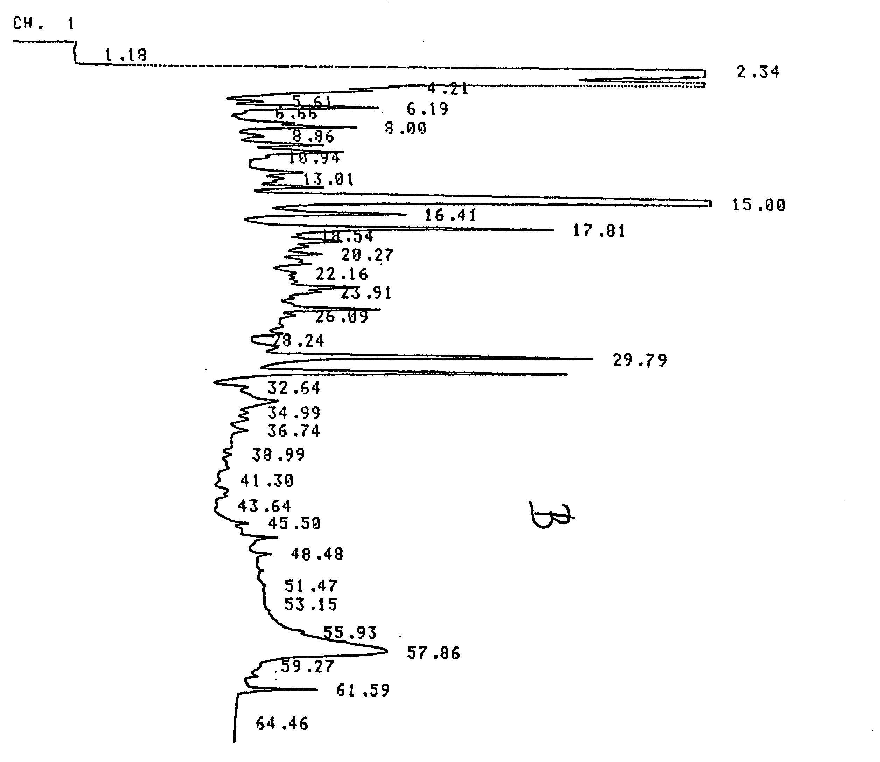

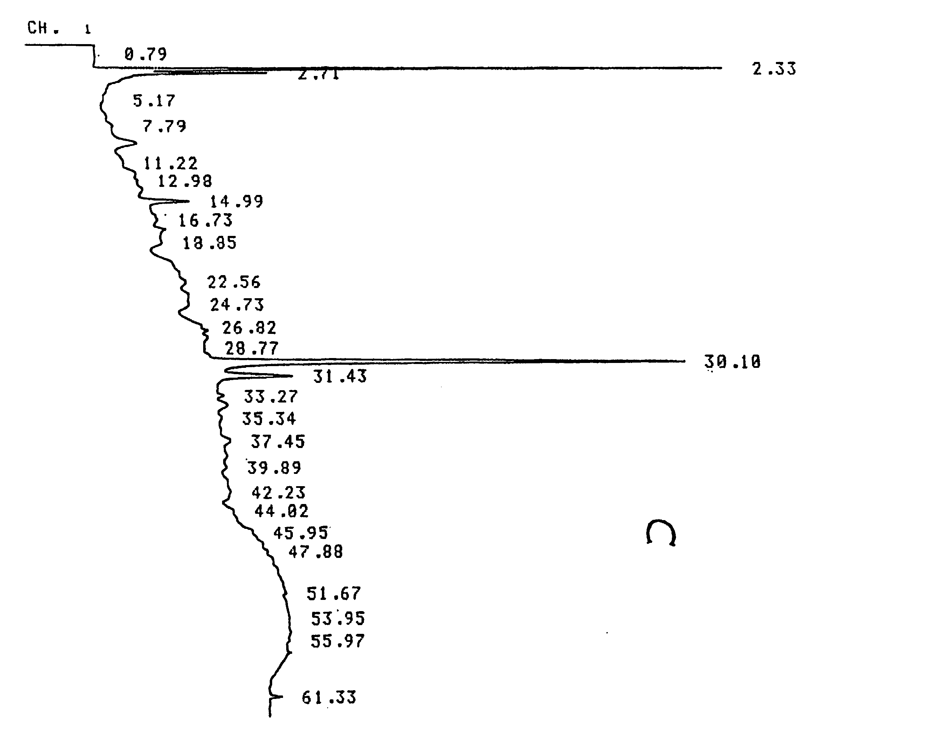

Die in Fig. 3 enthaltenen Chromatogramme zeigen:
A) Taraxacun (Blindprobe)
B) Taraxacum (Hauptversuch)
C) Taraxacum Freisetzung

Die Differenz zwischen Blindprobe und Hauptversuch zeigt, dass ca. 12 Substanzen gebunden sind, während die durch die Freisetzungsmethode nur 3 Substanzen ermittelt werden konnten.

## Patentansprüche

1. Verfahren zur Identifizierung mindestens einer aktiven chemischen Substanz aus einem Gemisch aktiver und inaktiver chemischer Substanzen mit **Molmassen von 150 bis 30.000, gekennzeichnet durch** die Schritte:
a) Hinzufügen eines Targets zu diesem Gemisch und Bildung eines Komplexes aus Target und mindestens einer aktiven chemischen Substanz des Gemisches, **wobei das Hinzufügen des Targets zu dem Gemisch von chemischen Substanzen in einer Lösung, einer Suspension oder einer Dispersion erfolgt,**
b) Abtrennung des Komplexes von den inaktiven chemischen Substanzen des Gemisches **mittels Filtration, Ultrafiltration, Zentrifugation, Ultrazentrifugation, Gleichgewichtsdialyse, Gelfiltration oder Präzipitation,** und
entweder
c) Freisetzung, Isolierung und identifizierung mindestens einer aktiven chemischen Substanz aus dem abgetrennten Komplex
oder
d) Identifizierung mindestens einer aktiven Substanz des Gemisches **durch** Differenzbildung eines Chromatogramms des Gemisches aktiver und inaktiver chemischer Substanzen und eines Chromatogramms des nach Abtrennung des Komplexes erhaltenen Gemisches inaktiver chemischer Substanzen.

2. Verfahren nach Anspruch 1, **dadurch gekennzeichnet, dass** Hinzufügen in einer wässrigen Lösung erfolgt.

3. Verfahren nach einem der vorangegangenen Ansprüche, **dadurch gekennzeichnet, dass** der pH-Wert der wässrigen Lösung mit Hilfe eines geeigneten Puffers stabilisiert wird.

4. Verfahren nach einem der vorangegangenen Ansprüche, **dadurch gekennzeichnet, dass** der Komplex durch eine Bindung zwischen der mindestens einen aktiven chemischen Substanz und dem Target hergestellt wird.

5. Verfahren nach einem der vorangegangenen Ansprüche, **dadurch gekennzeichnet, dass** es sich bei der Bindung um eine kovalente oder nicht-kovalente Bindung handelt.

6. Verfahren nach einem der vorangegangenen Ansprüche, **dadurch gekennzeichnet, dass** es sich bei der nicht-kovalenten Bindung um Wasserstoffbrücken, elektrostatische Wechselwirkungen, Metallkomplexierung, Wechselwirkungen von lipophilen Gruppen der aktiven chemischen Substanz mit dem Target, Dipol-Dipol Wechselwirkungen oder um Kation- Π-Wechselwirkungen handelt.

7. Verfahren nach einem der vorangegangenen Ansprüche, **dadurch gekennzeichnet, dass** die Isolierung und / oder Identifizierung der mindestens einen aktiven chemischen Substanz des abgetrennten Komplexes mit Methoden wie HPLC, Etektrochromatografie, Elektrophorese, Kopplungstechniken wie LC-MS oder MS-MS, vorzugsweise mittels Mikro-Kapillar- oder Nano-HPLC erfolgt.

8. Verfahren erfahren nach einem der vorangegangenen Ansprüche, **dadurch gekennzeichnet, dass** die Identifizierung der mindestens einen aktiven chemischen Substanz des Gemisches mit Methoden wie HPLC, Elektrochromatografie, Elektrophorese, Kopplungstechniken wie LC-MS oder MS-MS, vorzugsweise mittels Mikro-Kapillar- oder Nano-HPLC erfolgt.

9. Verfahren nach einem der vorangegangenen Ansprüche, **dadurch gekennzeichnet, dass** die Abtrennung der mindestens einen aktiven chemischen Substanz aus dem Gemisch mittels präparativer HPLC, Elektrochromatografie oder Elektrophorese erfolgt.

10. Verfahren nach einem der vorangegangenen Ansprüche; **dadurch gekennzeichnet, dass** das Gemisch aktiver und inaktiver chemischer Substanzen eine Substanzbibliothek, erhalten aus synthetischer oder kombinatorischer Chemie, oder ein Naturstoffextrakt ist.

11. Verfahren nach einem der vorangegangenen Ansprüche, **dadurch gekennzeichnet, dass** das Gemisch chemischer Substanzen ein chemisch modifizierter Naturstoffextrakt ist.

12. Verfahren nach einem der vorangegangenen Ansprüche, **dadurch gekennzeichnet, dass** das Gemisch chemischer Substanzen ein Gemisch verschiedener Naturstoffextrakte ist.

13. Verfahren nach einem der vorangegangenen Ansprüche, **dadurch gekennzeichnet, dass** das Gemisch aktiver und inaktiver chemischer Substanzen mindestens 50 verschiedene chemische Substanzen enthält.

14. Verfahren nach einem der vorangegangenen Ansprüche, **dadurch gekennzeichnet, dass** das Target ein Protein ist.

15. Verfahren nach einem der vorangegangenen Ansprüche, **dadurch gekennzeichnet, dass** das Target ein Enzym, ein Rezeptor, ein Antikörper, eine biologischen Membran oder eine Zelle ist.

16. Verfahren nach einem der vorangegangenen Ansprüche, **dadurch gekennzeichnet, dass** das Target das Enzym Thrombin ist.

17. Verfahren nach einem der vorangegangenen Ansprüche, **dadurch gekennzeichnet, dass** das Target das Enzym Trypsin ist.

18. Verfahren nach einem der vorangegangenen Ansprüche, **dadurch gekennzeichnet, dass** das Target der β₂-Adrenozeptor ist.

## Claims

1. Process for identifying at least one active chemical substance from a mixture of active and inactive chemical substances having relative molar masses of 150 to 30,000, **characterized by** the steps:
a) adding a target to said mixture and forming a complex of target and at least one active chemical substance of the mixture, this adding of the target to the mixture of chemical substances being performed in a solution, a suspension or a dispersion;
b) separating the complex from the inactive chemical substances of the mixture, by filtration, ultrafiltration, centrifugation, ultracentrifugation, equilibrium dialysis, gel filtration or precipitation, and
either
c) liberating, isolating and identifying at least one active chemical substance from the separated complex
or
d) identifying at least one active chemical substance of the mixture by subtracting from a chromatogram of the mixture of active and inactive chemical substances a chromatogram of the mixture of inactive chemical substances which is obtained after separation of the complex.

2. Process according to claim 1, **characterized in that** the addition is performed in an aqueous solution.

3. Process according to one of the preceding Claims, **characterized in that** the pH value of the aqueous solution is stabilized with the aid of a suitable buffer.

4. Process according to one of the preceding Claims, **characterized in that** the complex is created by a bond between the at least one active chemical substance and the target.

5. Process according to one of the preceding Claims, **characterized in that** the bond is a covalent or non-covalent bond.

6. Process according to one of the preceding Claims, **characterized in that** the non-covalent bond is formed by hydrogen bridges, electrostatic interactions, metal complexation, interaction of lipophile groups of the active chemical substance with the target, dipole-dipole interactions, or cation-π interactions.

7. Process according to one of the preceding Claims, **characterized in that** isolation and/or identification of the at least one active chemical substance of the separated complex is accomplished by methods such as HPLC, electrochromatography, electrophoresis, coupling techniques such as LC-MS or MS-MS, preferably by means of micro-capillary or nano-HPLC.

8. Process according to one of the preceding Claims, **characterized in that** identification of the at least one active chemical substance of the mixture is accomplished by methods such as HPLC, electrochromatography, electrophoresis, coupling techniques such as LC-MS or MS-MS, preferably by means of micro-capillary or nano-HPLC.

9. Process according to one of the preceding Claims, **characterized in that** the separation of the at least one active chemical substance from the mixture is performed by means of preparative HPLC, electrochromatography or electrophoresis.

10. Process according to one of the preceding Claims, **characterized in that** the mixture of active and inactive chemical substances is a substance library obtained from synthetic or combinatorial chemistry, or an extract of a natural product.

11. Process according to one of the preceding Claims, **characterized in that** the mixture of chemical substances is a chemically modified extract of a natural product.

12. Process according to one of the preceding Claims, **characterized in that** the mixture of chemical substances is a mixture of various natural product extracts.

13. Process according to one of the preceding Claims, **characterized in that** the mixture of active and inactive chemical substances contains at least 50 different chemical substances.

14. Process according to one of the preceding Claims, **characterized in that** the target is a protein.

15. Process according to one of the preceding Claims, **characterized in that** the target is an enzyme, a receptor, an antibody, a biological membrane or a cell.

16. Process according to one of the preceding Claims, **characterized in that** the target is the enzyme thrombin.

17. Process according to one of the preceding Claims, **characterized in that** the target is the enzyme trypsin.

18. Process according to one of the preceding Claims, **characterized in that** the target is the β₂-adrenoreceptor.

## Revendications

1. Procédé pour l'identification d'au moins une substance chimique active à partir d'un mélange de substances chimiques actives et inactives possédant des masses molaires de 150 à 30.000, **caractérisé par** les étapes consistant à :
a) ajouter une cible à ce mélange et former un complexe constitué par la cible et par au moins une substance chimique active du mélange, l'addition de la cible au mélange de substances chimiques a lieu en solution, en suspension ou en dispersion ;
b) séparation du complexe par rapport aux substances chimiques inactives du mélange à l'aide d'une filtration, d'une ultrafiltration, d'une centrifugation, d'une ultra-centrifugation, d'une dialyse d'équilibre, d'une filtration sur gel ou d'une précipitation, et soit
c) libérer, isoler et identifier au moins une substance chimique active à partir du complexe séparé, soit
d) identifier au moins une substance active du mélange par différenciation d'un chromatogramme du mélange de substances chimiques actives et inactives et d'un chromatogramme du mélange de substances chimiques inactives, obtenu après la séparation du complexe.

2. Procédé selon la revendication 1, **caractérisé en ce que** l'addition a lieu en solution aqueuse.

3. Procédé selon l'une quelconque des revendications précédentes, **caractérisé en ce qu'**on stabilise la valeur de pH de la solution aqueuse à l'aide d'un tampon approprié.

4. Procédé selon l'une quelconque des revendications précédentes, **caractérisé en ce qu'**on prépare le complexe via une liaison entre au moins une substance chimique active et la cible.

5. Procédé selon l'une quelconque des revendications précédentes, **caractérisé en ce que**, en ce qui concerne la liaison, il s'agit d'une liaison covalente ou d'une liaison non covalente.

6. Procédé selon l'une quelconque des revendications précédentes, **caractérisé en ce que**, en ce qui concerne la liaison non covalente, il s'agit de ponts hydrogène, d'interactions électrostatiques, de complexations de métaux, d'interactions de groupes lipophiles de la substance chimique active avec la cible, d'interactions dipôle-dipôle ou encore d'interactions du cation n.

7. Procédé selon l'une quelconque des revendications précédentes, **caractérisé en ce que** l'isolation et/ou l'identification de ladite au moins une substance chimique active du complexe séparé ont lieu avec des procédés tels que le procédé HPLC, une électro-chromatographie, une électrophorèse, des techniques de couplage telles que LC-MS ou MS-MS, de préférence à l'aide d'un procédé HPLC de type microcapillaire ou de type nano.

8. Procédé selon l'une quelconque des revendications précédentes, **caractérisé en ce que** l'identification de ladite au moins une substance chimique active du mélange a lieu avec des procédés tels que le procédé HPLC, une électrochromatographie, une électrophorèse, des techniques de couplage telles que LC-MS ou MS-MS, de préférence à l'aide d'un procédé HPLC de type microcapillaire ou de type nano.

9. Procédé selon l'une quelconque des revendications précédentes, **caractérisé en ce que** la séparation de ladite au moins une substance chimique active par rapport au mélange a lieu à l'aide d'une HPLC préparative, d'une électrochromatographie ou d'une électrophorèse.

10. Procédé selon l'une quelconque des revendications précédentes, **caractérisé en ce que** le mélange de substances chimiques actives et inactives est une bibliothèque de substances obtenues à partir de la chimie de synthèse ou de la chimie combinatoire, ou encore un extrait de substances naturelles.

11. Procédé selon l'une quelconque des revendications précédentes, **caractérisé en ce que** le mélange de substances chimiques est un extrait de substances naturelles soumis à une modification par voie chimique.

12. Procédé selon l'une quelconque des revendications précédentes, **caractérisé en ce que** le mélange de substances chimiques est un mélange de différents extraits de substances naturelles.

13. Procédé selon l'une quelconque des revendications précédentes, **caractérisé en ce que** le mélange de substances chimiques actives et inactives contient au moins 50 substances chimiques différentes.

14. Procédé selon l'une quelconque des revendications précédentes, **caractérisé en ce que** la cible est une protéine.

15. Procédé selon l'une quelconque des revendications précédentes, **caractérisé en ce que** la cible est une enzyme, un récepteur, un anticorps, une membrane biologique ou une cellule.

16. Procédé selon l'une quelconque des revendications précédentes, **caractérisé en ce que** la cible est l'enzyme thrombine.

17. Procédé selon l'une quelconque des revendications précédentes, **caractérisé en ce que** la cible est l'enzyme trypsine.

18. Procédé selon l'une quelconque des revendications précédentes, **caractérisé en ce que** la cible est le récepteur β₂-adrénergique.
